# EUROPEAN PATENT SPECIFICATION

(11) **EP 1 750 647 B1**
(45) Date of publication and mention of the grant of the patent: **19.11.2014**
(21) Application number: 05754113.8
(22) Date of filing: 23.05.2005
(51) Int. Cl.: A61K 9/20

(54) **PHARMACEUTICAL TABLETS HAVING A SEPARATION MARK POSITIONED ON THE SIDE OF SAID TABLETS**
PHARMAZEUTISCHE TABLETTEN MIT EINER TRENNMARKIERUNG AUF DER SEITE
COMPRIMES PHARMACEUTIQUES COMPORTANT SUR UNE FACE UNE MARQUE DE SEPARATION

(30) Priority: 21.05.2004 US 573042 P; 21.05.2004 US 573134 P
(43) Date of publication of application: 14.02.2007
(73) Proprietor: Accu-Break Technologies, Inc., Plantation, FL 33324 (US)
(72) Inventor: SOLOMON, Lawrence, Boca Raton, FL 33433 (US); KAPLAN, Allan, S., Boaca Raton, FL 33433 (US)
(74) Representative: Finetti, Claudia
(86) International application number: PCT/US2005/018638
(87) International publication number: WO 2005/112870

(56) References cited:
- EP-A1- 0 812 589
- US-A- 3 927 194
- US-A- 4 258 027
- US-A- 4 905 589
- US-A- 5 756 124
- DATABASE WPI Week 199410 Derwent Publications Ltd., London, GB; AN 1994-077223 XP002293384 -& JP 06 009375 A (TAKEDA CHEM IND LTD) 18 January 1994 (1994-01-18)
- VAN SANTEN E ET AL: "Breaking of scored tablets: a review" EUROPEAN JOURNAL OF PHARMACEUTICS AND BIOPHARMACEUTICS, ELSEVIER SCIENCE PUBLISHERS B.V., AMSTERDAM, NL, vol. 53, no. 2, March 2002 (2002-03), pages 139-145, XP004342806 ISSN: 0939-6411

## Description

### FIELD OF THE INVENTION

The invention describes segmented pharmaceutical tablets with markings positioned in or on said tablets in novel ways, preferably within one segment to direct optionally desired breaking of said tablets to be contained within one segment to aid in creating more accurate tablet breaking.

### BACKGROUND

Pharmaceutical tablets have long been provided with a score, which is a continuous indentation across a surface of the tablet. The purpose of the score is to locate a potential and desirable region of the tablet for the purpose of breaking (subdividing) said tablet into two smaller subunits, which are called tablettes herein.

Problems with current versions of scored tablets are well-known. These problems include loss of active drug and inaccurate division of the tablet, so that a tablet intended to be divided into two equal half-tablets often does not come close to that ideal.

It has thus long been recognized that it is difficult to break a tablet accurately, even if it is scored.

Many drugs require dosage adjustments, such as warfarin, the scored tablets of which are frequently broken. These dosage adjustments through tablet breaking by patients have been determined to be imprecise. As the following discussion demonstrates, for many years experts have called upon the pharmaceutical industry to improve the quality of tablet breaking, yet such has not been optimized until the current invention.

In 1984, Stimpel et al. ("Stimpel"), described the relative accuracy of breaking of various tablets for treatment of cardiovascular problems. M. Stimpel et al., "Breaking Tablets in Half." The Lancet (1984):1299. Even though breaking was performed by a sophisticated, dexterous person, Stimpel found that breaking was not accurate, and opined that real world use by patients would provide yet more unsatisfactory results. Stimpel called upon the pharmaceutical industry to improve the accuracy of splitting tablets: "Clearly any assumption that halving a tablet will hot lead to inaccurate doses is invalid. This potential source of inaccuracy could be even more significant in clinical situations (our study was done under ideal conditions) and the pharmaceutical industry should tackle it, either by improving divisibility (as already has been done for lopressor and logroton) or, even better, by marketing a wider range of unscored tablets to provide all the doses that might be indicated clinically."

Despite that finding and statement, and despite the issuance of various patents relating to optimizing a scoring pattern and/or tablet shape, Rodenhuis et al., (2004) noted that: "Improving the functioning of score lines may be a more practical approach than *banning* this dosage form" (emphasis added). N. Rodenhuis et al., "The rationale of scored tablets as dosage form." European J. of Pharmaceutical Sciences 21 (2004):305-308 (hereafter "Rodenhuis"). Rodenhuis observed that European regulatory authorities started a policy to discourage scoring of tablets in 1998. This policy change, according to Rodenhuis, likely related to "many recent reports of bad functioning score lines," that "many scored tablets are difficult to break," and that "many scored tablets show unsatisfactory mass uniformity of the subdivided halves." The authors then go on to describe useful aspects of scoring tablets. For a comprehensive review article on this topic, see van Santen, E., Barends, D.M. and Frijlink, H.W. "Breaking of scored tablets: a review." European J. of Pharmaceutics and Biopharmaceutics 53 (2002):139-145.

Some current studies that demonstrate the severity of the problem are described below.

Peek et al., (2002), studied tablet splitting by "elderly patients" aged 50-79. Peek, B.T., Al-Achi, A., and Coombs, S.J. "Accuracy of Tablet Splitting by Elderly Patients." The Journal of the American Medical Association 288 No.4 (2002):139-145. Breaking scored tablets with mechanical tablet splitters without specific instruction led to highly unsatisfactory separating of the tablets. For example, warfarin 5 mg was on average split into 1.9 and 3.1 mg tablets. This potent anticoagulant has such a narrow therapeutic range that 2, 2.5, and 3 mg tablet doses are manufactured. Biron et al., (1999), demonstrated that warfarin 10 mg also often split to less than 4.25 or greater than 5.75 mg. Biron, C., Liczner, P., Hansel, S. and Schved, J.F., "Oral Anticoagulant Drugs: Do Not Cut Tablets in Quarters." Thromb Haemost 1201 (1999). In addition, they demonstrated that loss of mass due to crumbling or chipping or the breaking of the warfarin tablets was statistically significant. They also demonstrated that quartering of the tablets was grossly inaccurate.

McDevitt et al., (1998), found that 25 mg unscored hydrochlorothiazide (HCTZ) tablets were manually split poorly enough that 12.4% deviated by more than 20% from ideal weight. McDevitt, J.T., Gurst, A.H., and Chen, Y. "Accuracy of Tablet Splitting." Pharmacotherapy 18 No.1(1998):193-197. 77% of the test subjects stated that they would be willing to pay a premium for individually produced HCTZ 12.5 mg tablets rather than split unscored 25 mg tablets.

Rosenberg et al., (2002), studied pharmacist-dispensed split tablets. Rosenberg, J.M., Nathan, J.P., and Plakogiannis, F. "Weight Variability of Pharmacist-Dispensed Split Tablets." Journal of American Pharmaceutical Association 42 No.2 (2002):200-205. They found that "tablet splitting resulted in an unacceptably high incidence of weight variation." They recommended that "standards should be developed to ensure uniformity of split tablets."

Teng et al., (2002), using a trained individual in a laboratory setting to split tablets, concluded that "the majority of the 11 drug products we tested, when assessed for their ability to be split into half-tablets of equal dose, failed a liberally interpreted USP (United States Pharmacopeia) uniformity test... The practice of dividing tablets to save costs or to improve a dosage regimen... is not recommended for patients using drugs with more substantial toxicity and steep dose-response efficacy curves." Teng, J., Song, C.K., Williams, R.L., and Polli, J.E. "Lack of Medication Dose Uniformity in Commonly Split Tablets." Journal of American Pharmaceutical Association 42 No. 2 (2002):195-199.

Rodenhuis reported that 31% of all tablets in one Netherlands study were subdivided before being swallowed. In the U.S., many "managed care" insurance organizations encourage splitting by patients of unscored, irregularly-shaped tablets. A great many drug products in the US are unscored or are provided as capsules despite being tablettable. The invention herein provides a solution for both scored and unscored tablets that provides an improved solution to the problems described above.

The Japanese patent document JP06009375 in the name of Takeda describes tablets which are made of subunits that are adhered together, using a cement or adhesive to form a whole tablet. Division of the tablets takes place by breaking through the adhesive layer disposed between subunits.

The United States of America patent US5756124 in the name of Invamed Inc. discloses in figure 2 a ring-shaped wider-than-tall tablet having grooves on its top and/or bottom surface as oriented in the tablet die.

The United States of America patent US3927194 in the name of Ives Laboratories Inc. describes in figure 1 a wider-than-tall tablet having a groove on one of its top and/or bottom surface as oriented in the tablet die.

The United States of America patent US4258027 in the name of Mead Johnson & Co. discloses in figures E, G and I a wider-than-tall tablet having two grooves on one of its surfaces as oriented in the tablet die.

The United States of America patent US4905589 in the name of Ackley Michael E. Relates to the marking of pellets using ink jet technology.

The European patent application EP812589 describes in example 3 a 3-layer wider-than-tall tablet composed of an active top and bottom layer separated by an inert layer, which is produced by granulation and subsequent compression.

In addition, treatment with combination products is common in pharmaceuticals, meaning that one dosage form may contain more than one active ingredient. This means of treatment may in part be discussed as follows:
A relevant field to the invention is the field of combination drug therapy for systemic arterial hypertension ("hypertension"). Technically, combination therapy for hypertension involves the use of two or more drugs on a regular basis to treat a patient's hypertension. Generally, this term implies daily treatment with at least two drug products.

Combination therapy has long been used to treat hypertension. It is widely estimated that approximately half of all cases of hypertension cannot be treated to goal blood pressure with one drug at a maximally tolerated dosage. To aid in treatment, solid oral dosage forms have been produced that contain a plurality of active agents within one tablet or capsule. These dosage forms are known as "fixed-dose" combination products, because a patient, nurse, or pharmacist has no means of separating one active agent from another. Sica (2002) states: "a considerable legacy, dating to the 1950's, exists for fixed-dose combination therapies." Later in the same article, the author pinpoints the deficiency with the fixed-dose approach, a deficiency which embodiments of the current invention provide novel and useful solutions for (Sica, D., Drugs, 2002; 62 (3)): "Rationale for Fixed-Dose Combinations in the Treatment of Hypertension:" "A disadvantage to the use of fixed-dose combination is a lack of dose administration flexibility for its individual components, although it is uncommon for physicians to maximally exploit the dose administration flexibility inherent to the use of free combinations. With fixed-dose combination therapy, if conditional amounts of either drug are required for BP control, a separate prescription will be required. This increases complexity of the regimen and has the potential to negatively affect compliance. In addition, fixed-dose combination therapy may not provide adequate drug amounts to manage illness, such as angina or congestive heart failure, which commonly co-exist with hypertension."

The invention provides a means to improve the above situation, by describing segmented, layered pharmaceutical tablets with novel locations of markings in or on them that aid in breaking the tablets in novel ways, preferably within one segment or layer rather through all segments or layers as is currently the practice with layered tablets.

It is not technically feasible to place a score solely in the middle layer of a three-layer tablet during high-speed manufacturing, as to do so would require a tablet die to have the equivalent of what on a punch is called embossing. A tablet formed with such a structure that extends from the die that would indent the tablet horizontally as it is formed in said die could in fact be created, but then it could not be ejected from said die, as to do so would destroy the tablet, the egress of the lower part of which would be physically blocked by said "embossing".

In addition to top and bottom scoring, it is known on occasion to create a vertical score that extends the height of the tablet, created by an "embossing" that is part of the tablet die. An example of a tablet so created is Desyrel Dividose®, which has a score created conventionally by embossing on the top or bottom punch as well as a vertical score running the height (short axis in this case) of the tablet, derived from an "embossing" arising from the die.

In addition, the use of other means of guiding a person to a breaking region that is preferably limited to one layer (segment) of a tablet that is composed of two or more layers has not been disclosed. Such potential means may include printing with edible ink, gelatin banding and perforations.

The current art of providing aids to breaking tablets comprising a plurality of layers is exemplified by products with the trade name of Norgesic® and Norgesic Forte®. This three-drug product was produced in three layers, one formed solely with aspirin, one solely with caffeine, and the other solely with orphenadrine citrate. A top layer (or, alternatively, bottom layer) score provided limited superficially to said layer, and was located at the middle of said layer. Breaking the tablet in a conventional way through the score would bring the break through all three layers of the tablet, with the clear intent that the break 5 be into halves for each of the three layers. Until the current invention, it has not been taught to provide an aid to locate a breaking point in the middle layer of such a three-layer tablet so that such a tablet could be broken to provide two smaller tablets ("tablettes" herein), one containing the entirety of the top layer and a portion of the middle layer, and one containing the bottom layer and a portion of the middle layer.

### SUMMARY OF THE INVENTION

The invention provides a segmented layered pharmaceutical taller than wide tablet obtainable by sequential introduction of granulations into a tablet die, having a separation mark positioned within a segment which is not a top or bottom segment, and which lacks a pharmacologically active quantity of any drug; such that on application of force the tablet will break through the inactive part of the tablet, wherein the separation mark or marks is/are selected from the group consisting of one or more of the following:
(a) a score located in a side wherein said score is not oriented vertically and is preferably oriented horizontally; (vertical and horizontal orientation may be determined
   according the present disclosure)
(b) indicia on at least one side that locates a desired breaking region of said tablet;
(c) a band which is located on one segment.

A preferred pharmaceutical tablet according to the invention is one where a separation mark is positioned in a single segment which is not a top or bottom segment.

The invention also includes pharmaceutical tablets where at least one additional separation mark is placed in one or more segments which is a top or bottom segment.

Accordingly, it is a primary object of the invention to provide a separation mark that locates a region of a tablet and directs breaking through less than all segments.

It is also an object of the invention to provide a separation mark that provides a guide to breaking a tablet through a relatively inactive segment.

It is also an object of the invention to provide an improved method of creating tablet parts by breaking a tablet in such a manner that increases the accuracy of breaking of both scored and unscored tablets by providing a tablet having a relatively inactive part and directing the breaker of the tablet to apply force to cause such a tablet to break through such a relatively inactive part of the tablet.

These and other objects of the invention will become apparent from the present specification.

### BRIEF DESCRIPTION OF THE SEVERAL VIEWS OF THE DRAWINGS

Fig. 1a is a cross-section of a taller than wide tablet looking towards the side of the tablet having a score.
Fig. 1b is a cross-section of the tablet of Fig.1a looking at the side of the tablet where the score ends.
Figs. 2a-d are views of Fig 1a and Fig. 1b respectively when the tablets have been broken through the score;
Fig. 3 is a cross-section of a taller than wide tablet having two segments, one of which is about three-quarters of the length of the tablet;
Figs. 4a-b are views of Fig. 3 when the tablet has been broken at the approximate mid-point of the tablet.
Fig. 5 is a cross-section of a taller than wide tablet having five segments;
Figs. 6a-b are views of Fig. 5 when the tablet has been broken through one segment;
Figs. 7a-c are views of Fig. 5 when the tablet has effectively been broken through two segments in two steps, first by breaking the tablet and then by breaking the tablette of Fig. 6b;
Fig. 8 is a perspective view of a scored tablet according to the invention;
Fig. 9 is a front view of a banded capsule according to the invention;
Fig. 10 is a front view of a tablet having perforations in the surface according to the invention; and
Fig. 11 is a front view of a tablet having two printed dotted lines on the surface according to the invention.

### DETAILED DESCRIPTION OF THE INVENTION

The separation marks by themselves will suggest the application of breaking forces that will cause a break at the separation mark as in the case of conventionally scored tablets. However, it is also contemplated that written instructions that explicitly call attention to the separation marks may be included in the packaging or labels of a pharmaceutical product which utilizes the present invention.

The term "segment" is utilized herein to describe the tablets of the invention. A segment represents the entirety of a substantially homogeneous contiguous region of a tablet or tablette (see below) of the invention. A segment is derived from one or more layers. A layer is formed after a granulation has completely entered a die of a tablet press (i.e, a "fill" has occurred). In a compressed tablet, which is the main topic of the invention, a layer is considered to be present whether said granulation is untamped, tamped, or fully compressed. Two or more consecutive feeds of substantially identical granulations may be utilized in forming the tablet, when these two substantially compositionally identical layers are compressed, they will generally be treated as one unit of the tablet. This "unit" is called a segment, more specifically a compound segment. A segment comprises one layer if said layer is contiguous (undivided) and is not contiguous to a substantially identical layer; such a segment is called herein a simple segment. A segment formed from a plurality of contiguous substantially identical layers is called a compound segment herein.

As used herein, such terms as "horizontal" ("transverse") and "vertical" when used in relation to a tablet, are based on the spatial orientation of the tablet as, and after, it is produced in a die, but before it is removed or ejected from the die. Thus, the top layer (segment) of the tablet is considered to be above the inner and bottom layers (segments). The layers and segments of the tablet are considered to be vertically disposed with regard to each other, as granulations enter and form layers vertically.

The height of a tablet represents the vertical distance from the lowest part of the tablet to the level of the highest part. There are two transverse dimensions, measured by taking a cross-section of the tablet through its widest part. Unless the cross-sectional configuration of the tablet is circular or square (excluding beveling or cupping at the periphery of an otherwise square cross-sectional shape), then there are at least two different transverse dimensions. The greater of said two transverse dimensions is called the width, and the lesser is called the depth. Tablets of the invention comprise at least two segments, have release characteristics that are immediate release (i.e., there are no controlled-release or "drug delivery" coatings, additives, or characteristics), and have a height that exceed the width. Tablets of the invention are not formed using a cement, glue, adhesive, or the like.

In the case of a segment that contains active drug in a diluted concentration relative to another segment that contains said drug, or in the case of a segment that contains a concentration of a drug that is the maximal concentration of said drug in any segment of the tablet (where greater precision of breaking is even more beneficial), the greater is the need for the separation mark to be limited in size or to point to an increasingly limited region in scope, so that tablet breaking can reliably occur in the most desirable location of the tablet.

The major separated structures produced by breaking tablets of the invention are called tablettes herein and in preferred embodiments they will contain precise amounts of a drug or drugs.

Examples of separation marks include: (a) a score, having any type of profile such as triangular or square, in one or more surfaces of the tablet; (b) printed indicia such as a solid or dotted line at a desired breaking region on the tablet; (c) printed indicia such as a logo, the word "break" or the words "break here" with or without a symbol suggesting breaking such as a representation of a scissors, an arrow or a dot or series of dots; (d) indentations may be arranged in a punctuate but linear fashion across a transverse dimension of the tablet in a suitable pattern; (e) a band such as a tape which encircles all or a part of 5 the tablet such as a gelatin band or ingestible polymeric tape; (f) perforations through a tablet, which may without limitaton be produced by mechanical removal of a part of the tablet to depth of e.g. 0.1 to 0.5mm or by a laser having an effective wave length and power to remove the desired amount of material and may be arranged in a suitable pattern;

All of these may aid in locating a desired breaking region, and may aid the physical act of breaking tablets of the invention as desired. Thus a preferred separation mark is a score that runs horizontally across an inner segment of a three-segment tablet, in which said inner segment lacks a pharmacologically active quantity of any drug and both outer (top and bottom) segments contain pharmacologically active quantities of the same or different drugs.

A score of the above type cannot be produced during commercial tablet manufacturing because to do so would require a horizontal "shelf" or "embossing" in the die that would allow tablet formation but would prevent ejection of the formed tablet from the die. Such a score may however be produced by other means, such as by use of a file manually or by a high-speed manufacturing process. Various methods of applying indicia such as pharmaceutically acceptable inks and banding materials are well known and may be applied to the side of the tablet to locate a region of potentially desired tablet breaking, such as through a relatively inactive segment which is interposed between two segments.

The depth of a score may be from about 0.5mm to about 3mm but should not exceed from about 40-50% of the thickness of the tablet. The length of the separating mark is not critical and in the case of a rounded tablet may include 30° to 180° of arc or more preferably from 60 to 120° of arc; in the case of a rectangular, trapezoidal etc. tablet, the score may include from about 10% to 50% percent of the perimeter of the tablet measured at the point where the separation mark is placed.

The terms "active agent," "active drug," "drug," "active pharmaceutical ingredient" and "pharmacologically active agent" are interchangeable herein and refer to a chemical material or compound which, when administered to an organism (human or animal) induces a pharmacological effect, and which includes prescription and non-prescription pharmaceutical compounds, pharmacologically effective amounts of such substances as vitamins and cofactors. Not considered as a "drug" are such substances as foodstuffs or vitamins in "recommended daily allowance" amounts.

Pharmaceutical tablets of the invention are produced in a layered fashion, utilising sequential introduction of granulations into a tablet die. For specificity, tablets are described in terms of top, bottom, sides, vertical dimension (or axis), horizontal '(transverse) dimension (or axis) in terms of their order of formation in the die. Thus, a tablet formed from sequential introduction of three different granulations into a dire will form a tri-layer tablet, describable as follows: The first' granulation in the die forms the bottom layer (which is an outer layer); the second granulation forms an inner layer; the third and last granulation forms the top layer (which is an outer layer). The theoretical axis running from the bottom of the tablet to the top is the vertical axis, and is denoted as the vertical direction of the tablet, without regard to the orientation of the tablet after it leaves the die. A. line or plane perpendicular to the vertical axis or dimension is a horizontal or transverse line or plane.

The main object of the invention is to allow convenient breaking of the tablet typically through a single segment; a preferred region for breaking is at a segment lacking a pharmacologically effective dose of any drug.

If separate granulations were able to be sequentially placed in a die horizontally (side-to-side) and not vertically as is currently the practice, then the tablets so produced would be within the scope of the present invention, as the same product would be produced.

When force is applied to break the tablet at or about the separation mark in a direction that is substantially perpendicular to the surface on which it is desired that breakage of the tablet will be initiated, breaking of the tablet produces predictable quantities of active ingredient(s). The tablet may be broken according to the invention either by applying force such as a cutting edge directly to the separation mark, or to other areas of the tablet, such as the outer segments, to cause the tablet to break at or about the separation mark and in the direction of the separation mark.

Separation marks are intended to guide optional tablet breaking in the usual manner that is well known with scores, so that force will be applied to break the tablet at or about the separation mark in a direction that is substantially perpendicular to the surface on which it is desired that breakage of the tablet will be initiated. The tablet may be broken according to the invention either by applying force such as a cutting edge directly to the separation mark, or to other areas of the tablet, such as the outer segments, to cause the tablet to break at or about the separation mark and in the direction of the separation mark.

The drawings depict vertical cross-sectional views of tablets and tablettes of the invention. Tablets are depicted as if they were in the die, so that the top of the tablet as it is oriented on the page corresponds with the top of the tablet in the die. In other words, the top segment of the tablet as viewed contains the last granulation to enter the die. Tablettes are depicted as they would have been in the die before they were separated from the intact tablet.

"Front views" refer to a cross-sectional view of a tablet that has a theoretical geometric plane passed through the tablet relative to a side which is arbitrarily designated as the front. Figures labeled as "side view," which also have a corresponding "front view" are taken as a cross-section through the whole tablet from the right side of a front view i.e., a side view is a cross-section that is taken by passing a plane through the vertical axis of the whole tablet at a 90° angle to the cross-sectional front view. Each front view represents a schematic cross-section that passes through the midpoint of the horizontal cross-section as measured from the front of the tablet to the back of the tablet or tablette. The front view is also parallel to the major axis of the tablet (e.g., for a tablet with a rectangular (but not square) transverse cross-section, the longer side of the perimeter is parallel with the plane that depicts the cross-sectional, front view. That plane is located half-way between the front and back surfaces of said tablet.

Segments containing a pharmacologically effective amount of a drug are shown crosshatched; segments lacking any drug or lacking a pharmacologically amount of any drug or drugs are shown plain (clear, without crosshatching or stippling). The upper part of each figure corresponds to the upper part of a tablet, all of which are depicted as they are situated within a die after final compression and before ejection from the die. For consistency, tablettes are depicted in the same orientation as the tablets from which they are formed, although tablettes are created after tablet formation. Separation marks in the tablets depicted in the figures are depicted as scores that are present on or in the surface of the tablet and that do not extend deeply enough into the tablet to appear in the cross-sectional front views are depicted in the drawings as dotted lines to reflect the location of said scores on or in the surface of the tablet (not shown). It is to be understood that the depth of a separation mark or other score may be deeper than one-half the widest cross-section of the tablet in a particular embodiment, and thus the transverse dotted lines reflecting surface scores that are separation marks imply no intention to limit the depth of any scores of the tablets of the invention. Similarly, a dotted line that reflects a surface score does not limit the vertical or horizontal extent, or height or depth of scores that serve as separation marks. Perforations or discontinuous scores are within the scope of the invention. A perforation may be produced by a laser of suitable power and wavelength, or by a thin instrument. A perforation herein defines a hole such as a "tunnel" that extends from one side of a tablet to another side, preferably an opposite side. Any scores or printed indicia that serve as separation marks are for convenience herein assumed to be on the front surface of the tablet, which is arbitrarily chosen from a vertically-oriented surface of the tablets. The "side view" of a tablet is a cross-sectional view of the tablet rotated 90 degrees from the front view. No dimension of separation marks is limited by their depiction as dotted lines in any Figure. In addition, no limitation of the usefulness of such indicia as printed separation marks to guide breaking in intended. Tablettes are depicted with broken surfaces as indicated by a fine saw-tooth pattern. Such saw-tooth depiction is schematic and not intended to represent the actual pattern of breaking of a tablet or tablette.

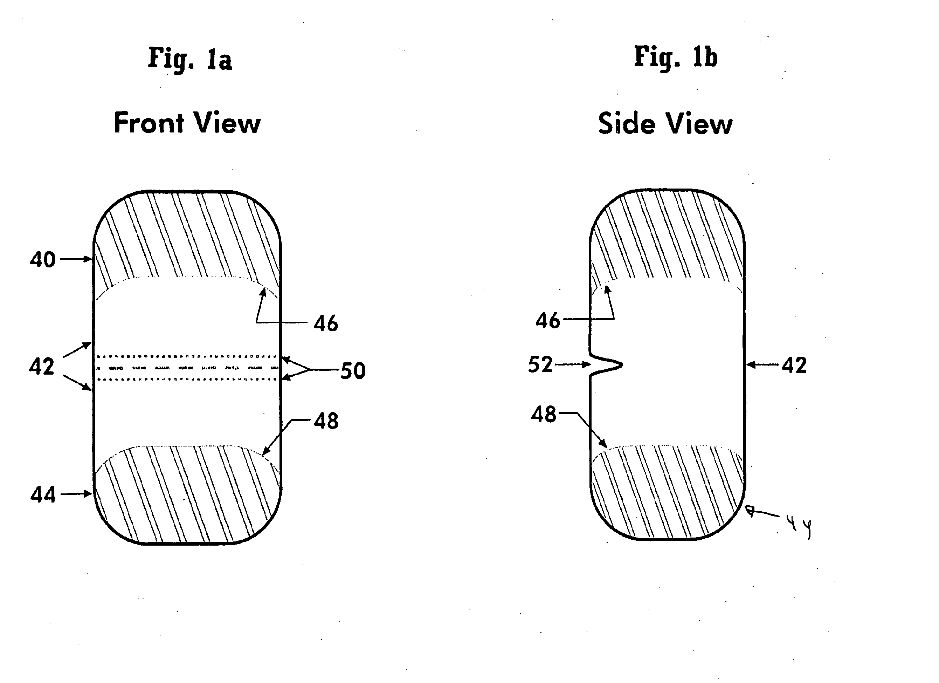

Figures 1a and 1b depict a tablet with compositionally substantially identical upper segment 40 and lower segment 44. Inner segment 42 contains trace amounts of the drug that is present in a therapeutically effective quantity in each of segments 40 and 44. Interfaces 46 and 48 represent regions in which the upper part of segment 42 and the lower part of segment 42 respectively adjoin upper segment 40 and lower segment 44. The curved interfaces result from the profile of the upper tablet punch which is curved. Score 52 is depicted in Fig. 1b. Dotted line 50 in Fig. 1a is a reflection of score 52 on the surface of the tablet (not shown), that does not penetrate half-way through the shorter transverse axis of the tablet.

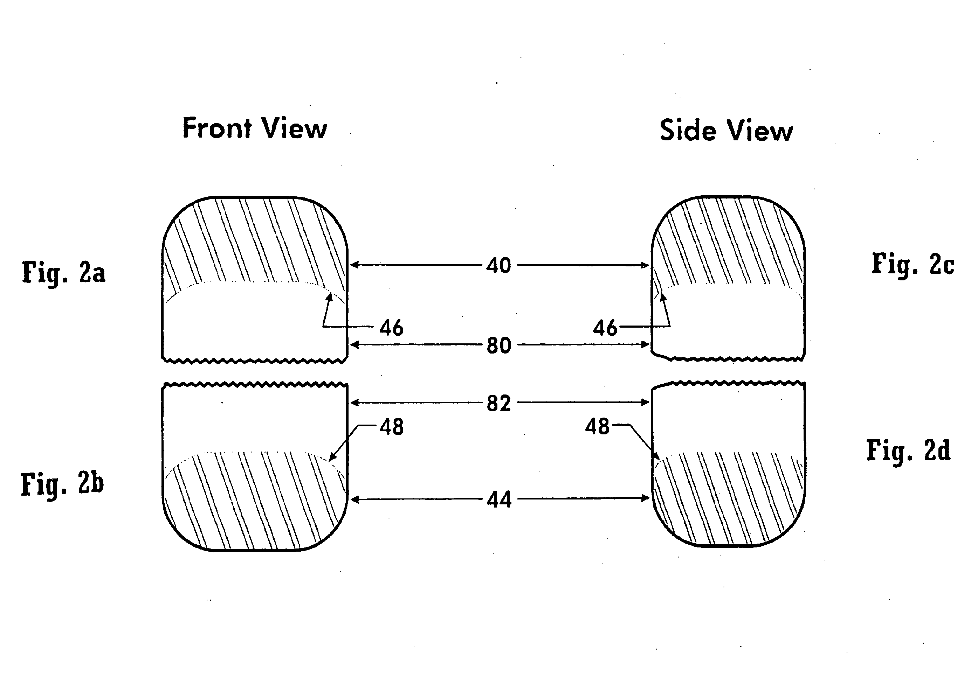

Figs. 2a-d depict tablettes formed from breaking the tablet' of Figs. 1a and 1b through score 52. Inner segment 42 of Fig. 1a no longer exists as an intact segment. The upper tablette of Figs. 1a and 1c contains segment 80 attched to an intact upper segment 40 and the lower tablette contains segment 82 and intact segment 44.

Breaking the tablet of Fig. 1a and 1b through the score placed in segment 42 is clearly easier than breaking the tablet through its vertical dimension, which is currently the practice with scored layered (segmented) tablets. The fact that no break is made in the parts of the tablet where the active drug has been placed provides for exceptionally accurate breaking relative to the active drug or drugs contained in the tablet.

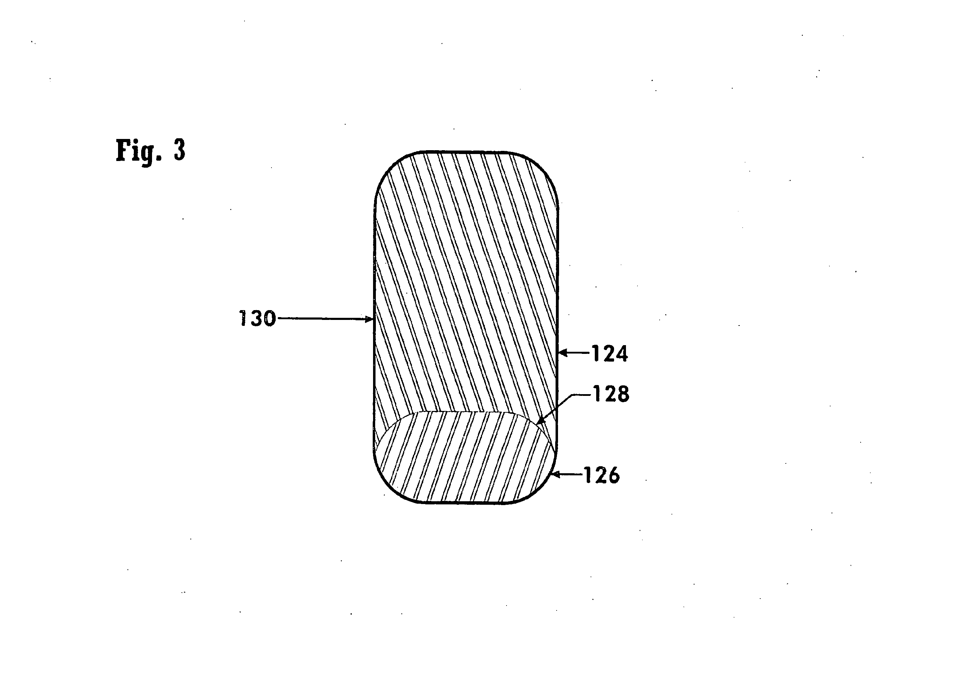

Fig. 3 demonstrates a two-segment tablet, each segment formed from a granulation containing a pharmacologically effective amount of medication. Upper (outer) segment 124 is larger than lower (outer) segment 126. Interface 128 indicates a region at which said segments are contiguous. A printed mark on the outer surface of the tablet (not shown) indicates a desired breaking point, as indicated by the location of arrow 130 that reflects the position of said surface printed mark. The two segments also have different color, however, further allowing identification of which part of the tablet contains which segment.

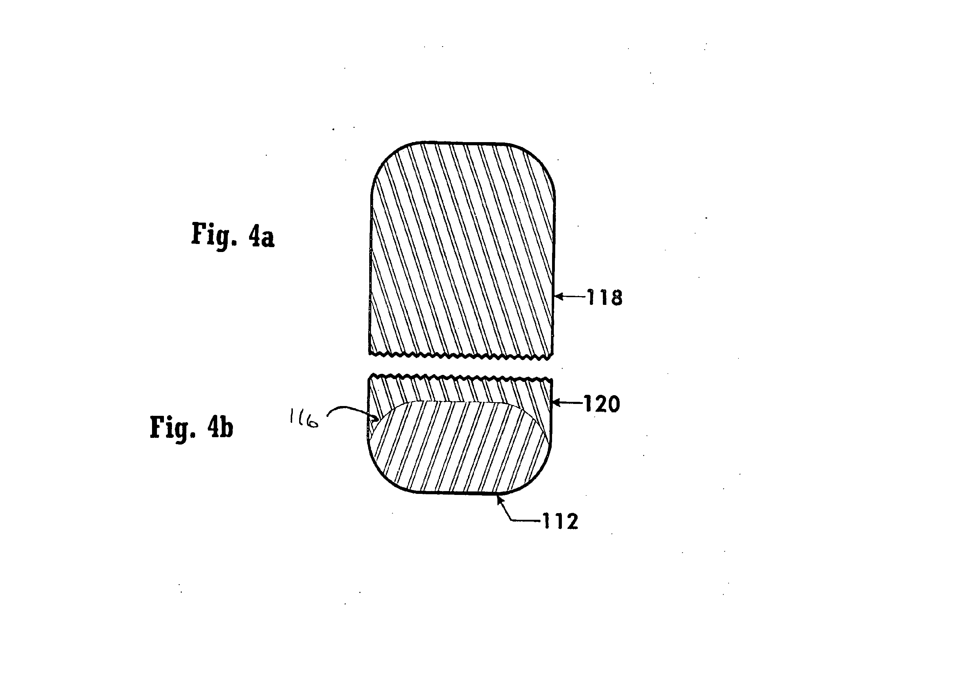

Figs. 4a and 4b depict the two tablettes formed by breaking the tablet of Fig. 3. The tablette of Fig. 4a consists of segment 118, which represents the bulk of segment 124 of Fig. 3. The tablette depicted in Fig. 4b contains segment 112 in an intact form and segment 120, which represents a less than half-portion'of segment 126 of Fig. 3. Interface 116 indicates a region at which said segments are contiguous. The curved face is due to the profile of the tablet punch.

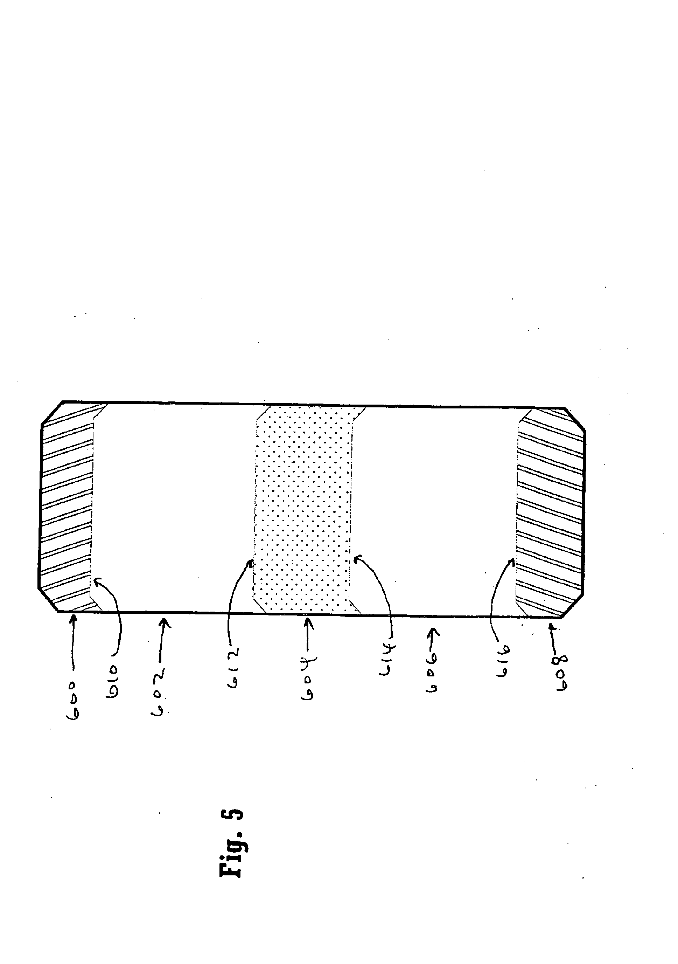

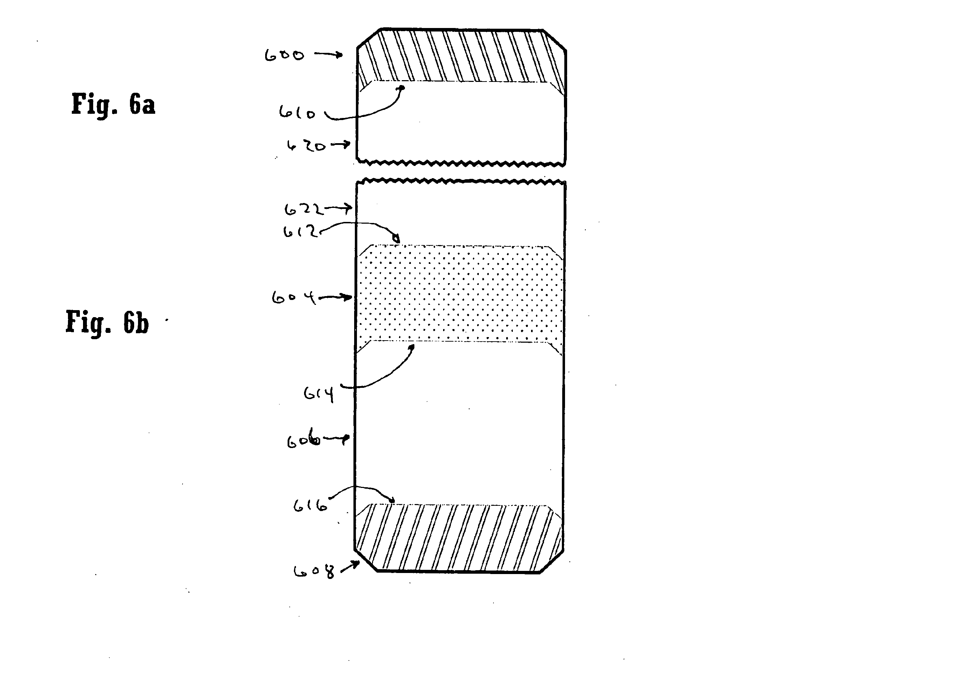

Fig. 5 which is outside the scope of the claims illustrates a tablet more elongated than those previously demonstrated. This tablet is adapted, even more than the others, for ease of breaking through one segment. Upper segment 600 is provided with a therapeutic quantity of a drug; stippled inner segment 604 is provided with a therapeutic quantity of a different drug; and, lower segment 608 is provided with a therapeutic quantity of a drug different from that found in a therapeutic quantity in segments 600 and 604. Clear (plain) inner segments 602 and 606 contain pharmacologically ineffective amounts of each of the three drugs found in the tablet. Interfaces 610, 612, 614, and 616 represent the regions at which two contiguous segments adjoin. The tablet of Fig. 5 is provided with a different color for each segment. Even though there is no surface scoring or indicia, the color scheme is such that a person's attention may be directed to apply force to break the tablet through segment 602 to create the tablettes depicted in Figs. 6a and 6b. Fig. 6a depicts the smaller tablette created by breaking the tablet of Fig. 5 through segment 602 in a transverse fashion. Segment 620 has been created by said breaking, and segment 602 of Fig. 5 no longer exists as a intact segment. Fig. 6b depicts the larger tablette created by said breaking of the tablet of Fig. 5. New upper segment 622 has been created.

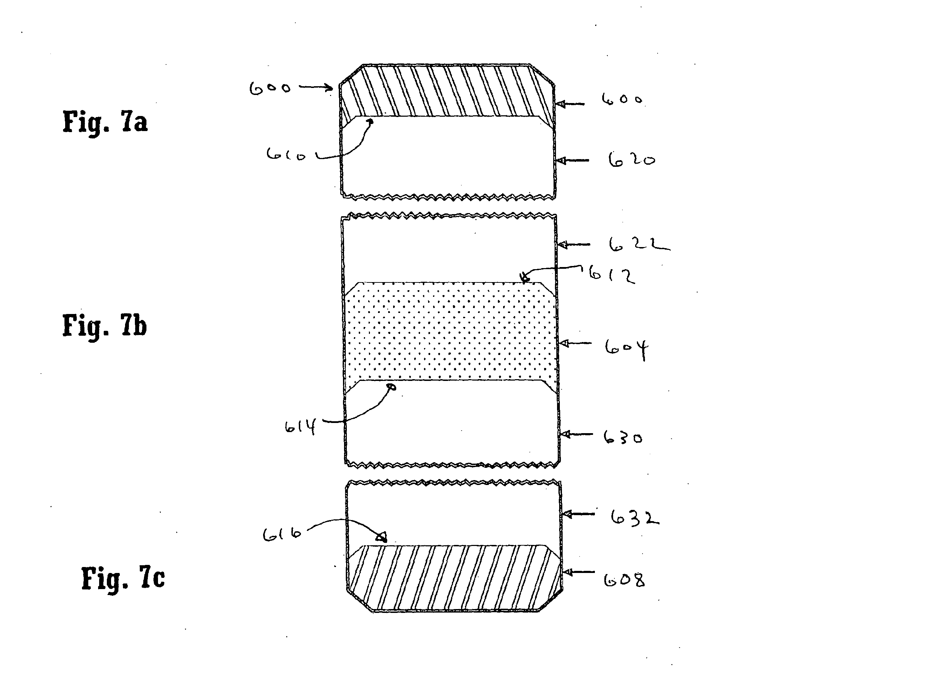

Figs. 7a-c depict three tablettes created by the subsequent breaking of the tablette of Fig. 6b. New segment 630 and segment 632 have been created and segment 606 no longer exists as an intact segment.

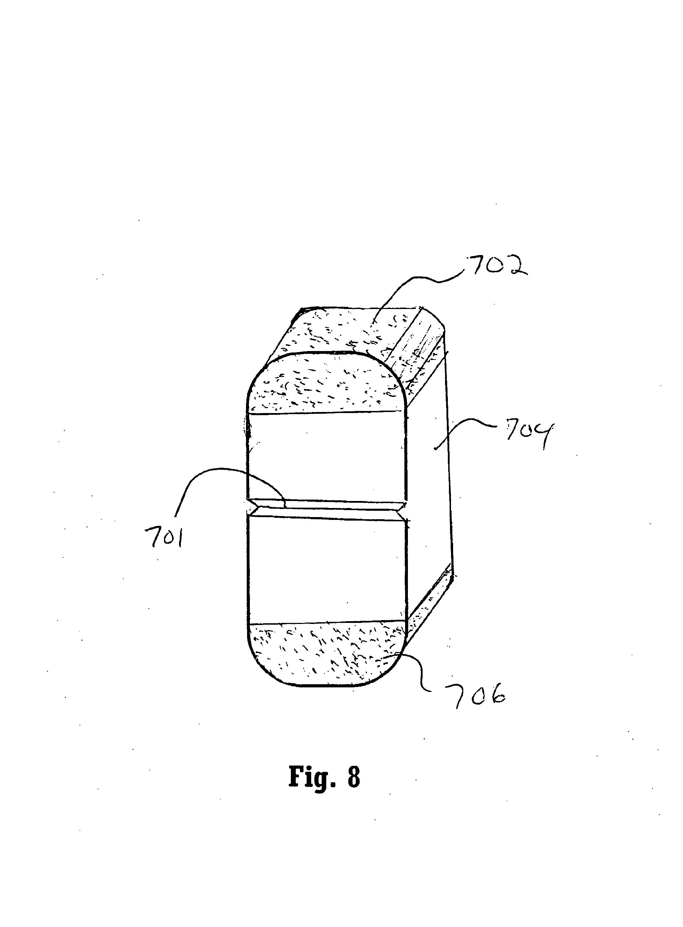

Fig. 8 is a perspective view of a tablet of the invention which shows score 701 as a separating mark on a front surface and top active (drug containing) segment 702; middle inactive segment (no detectable drug or a pharmacologically ineffective amount of a drug) and bottom active segment 706. When the tablet is broken at the score 701, the top segment and the bottom segment will remain intact.

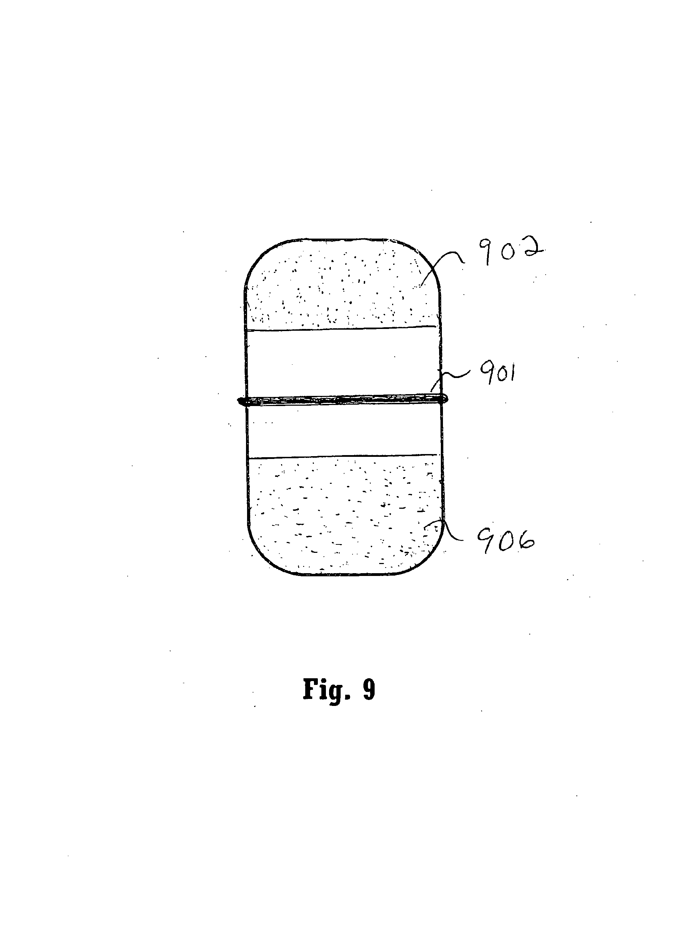

Fig. 9 is a front view of a tablet of the invention showing a band 901, such as a gelatin band that is used to seal hard gelatin capsules, which is applied to suitable tablets according to the invention to provide a separating mark. Techniques such as those use to band capsules, as disclosed in U.S. 4,922,682, which is incorporated by reference, may be modified to provide a band in making tablets according to the invention.

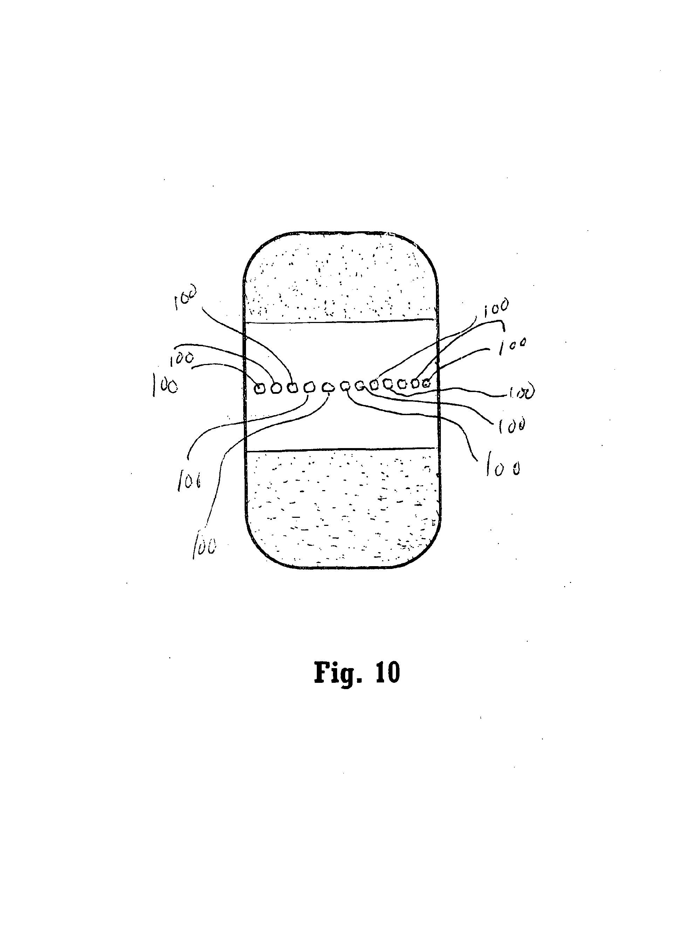

Fig. 10 shows a series of perforations 100 that may be made in the surface of a tablet to form a separation mark according to the invention. These perforations may be formed e.g. by mechanical or laser drilling 1-2mm holes that extend into the surface to a depth of 1-2mm.

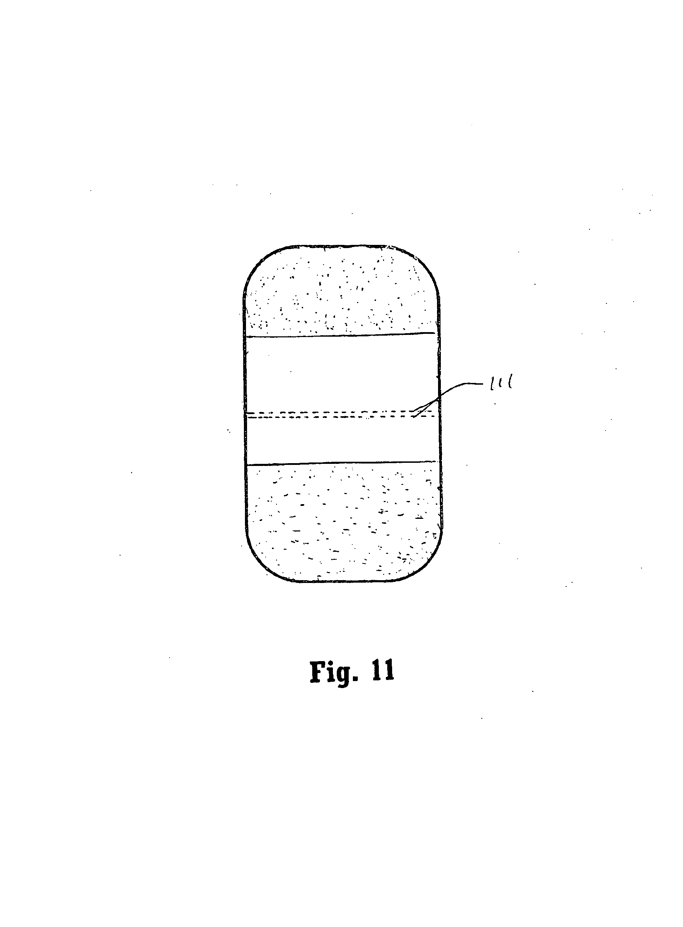

Fig. 11 shows a front view of a tablet according to the invention that has two printed dotted lines that serve as a separation mark according to the invention.

Granulations may be produced according to formulae involving or lacking active drug. A granulation lacking an active drug is an inactive granulation herein.

After production of a tablet from more than one non-identical granulation, tablets of the invention undergo novel further treatments. A preferred embodiment is as follows, but is not limiting:
A first granulation comprising amlodipine (a cardiovascular agent) enters a die, followed by an inactive granulation, followed by. a substantially identical granulation to the first granulation. After tablet compression and ejection from the die, the tablet is manipulated and a score is 5 placed into the middle section, which is derived from the inactive granulation. Scoring may be done manually, semiautomatically, or automatically Alternatively, a line transversely across said middle segment may be created by application of ink, by techniques well known in the art, or by application of edible tape. As a further alternative, tablets may be manipulated and subjected to laser energy so that one or more through-and-through perforations are created in the middle segment. All of these techniques place a separation mark in or on one segment, so that tablet breaking may be accurately undertaken to create predetermined amounts of active drug in each broken part of the tablet; such "halves" are called tablettes herein. The invention thus solves the problem much discussed, which is that tablets, whether or not they are scored, do not reliably break into even parts when desired. The invention does hot require that tablet breaking be into equal halves when force is applied to a middle segment derived from an inactive granulation, but succeeds in its object by in the above example, placing the high concentrations of active drug in different regions from the region of breaking.

Other methods may be utilized according to the invention to help achieve the object of the invention. In the above example, the top and bottom segments could be whitish, and the middle segment could be green. The green middle segment could thus be considered as a separation marking, as it locates a desired region of breaking of said tablet when tablet breaking is recommended.

Another embodiment involves introduction of granulations with different drugs into the top and bottom segment, as follows:
A granulation comprising hydrochlorothiazide (HCTZ) enters the die, followed by an inactive granulation, followed by a granulation comprising ramipril. After the three-segment (and three-layer) tablet is compressed and ejected from the die, the tablet is scored through the middle segment. Thus, application of force such as by hand or with an instrument such as a knife or commercially available tablet splitter can easily cause the tablet to break (such as is shown in Fig. 3) into two tablettes, one containing a therapeutic dose of HCTZ, the other containing a therapeutic dose of ramipril, both tablettes containing a part of the middle segment. Whether the desired breaking point is via a score, printed or other indicia, or perforations is less important than the fact that the invention provides visual (and, in the case of a score or perforations, and potentially via edible tape), tactile information sufficient to allow one to break the tablet through the proper segment so as to achieve the desired result.

Tablets of the invention are not limited to any specific number of drugs. They need not contain a segment derived from an inactive granulation. For example, a two-segment tablet may be created as follows:
Two successive substantially identical granulations comprising a microencapsulated potassium chloride preparation enter the die, followed by a granulation comprising furosemide (a diuretic). Said tablet therefore is a two-segment tablet, namely a simple segment of furosemide and a compound segment of KC1. After final compression of the layers and ejection of the tablet from the die, the tablet may be scored in various places, such as horizontally (transversely) through the KCl segment. The tablet can be utilized whole as a combination product, or can be broken through the score. The tablettes could each be useful; a tablette composed of furosemide and some amount of KCl less than the amount in the whole tablet could be of therapeutic value, and an amount of KCl substantially lacking furosemide could also be of therapeutic value.

Some specific uses of treating patients or other humans or other animals in need thereof follow subsequently. Some of the great many pharmaceutical agents that could be usefully provided in tablets according to the invention are listed subsequently.

In addition, the invention is useful in providing a combination tablet in a fashion that substantially allows one active agent to be separated from another. Some of the many products that could usefully be created according to the invention are described subsequently.

### DESCRIPTION OF MANUFACTURING PREFERRED EMBODIMENTS

A taller than wide tablet is made which has three segments, each with an active top or upper segment and an active lower or bottom segment separated by a substantially inactive middle segment. A Stokes 27-station tri-layer rotary tablet press is used. All formulations are directly compressible powder blends. The blending both of the amlodipine formulation and the benazepril formulation are performed in a Patterson-Kelly "V" blender. The middle segment consists of 194 mg of Nu-Tab® and requires no blending. The tablets are compressed using 0.131 inch by 0.3222 inch oval, concave tablet punches to a hardness of 35 kiloponds. The bottom segment is introduced first into the die. The tablet weight is 310 mg. Tablets so made are 8 mm tall; the inactive middle segment varies from 5-6 mm in height and a width of 4mm.

Weights in mg of the granulation comprising each segment are as follow:

| Bottom Segment | Mg. |
|---|---|
| Dibasic calcium phosphate anhydrous | 51.13 |
| Amlodipine besylate | 7.15 |
| Sodium starch glycolate (Explotab®) | 2.48 |
| Magnesium stearate | 0.93 |
| FD&C Blue #1 Aluminum Lake | 0.31 |
| Total | 62.00 |

### Manufacturing Instructions

1. Weigh each ingredient.
2. Screen each ingredient.
3. Triturate the color with the major diluent in geometric proportions using a suitable mixer.
4. Add the remaining ingredients, except the lubricant, to the color mixer from step #3 and mix for desired time.
5. Add the lubricant to the blend from Step #4 and mix for desired time.
6. Add the blend to a suitable press fitted with the desired tooling and compress into tablets.

| Middle Segment | Mg. |
|---|---|
| Nu-Tab® (Compressible sugar 30/35 N.F.) | 194.00 |

| Top Segment | Mg. |
|---|---|
| Lactose 310 monohydrate | 42.03 |
| Benazepril HCl | 9.00 |
| Crospovidone | 2.16 |
| Magnesium stearate | 0.54 |
| FD&C Red #40 Aluminum Lake | 0.27 |
| Total | 54.00 |

### Manufacturing Instructions

1. Weigh each ingredient.
2. Screen each ingredient.
3. Triturate the color with the major diluent in geometric proportions using a suitable mixer.
4. Add the remaining ingredients, except the lubricant, to the color mixer from step #3 and mix for desired time.
5. Add the lubricant to the blend from Step #4 and mix for desired time.
6. Add the blend to a suitable press fitted with the desired tooling and compress into tablets.

### Tabletting Instructions

1. Place the powder for active layer in hopper #1.
2. Place the powder for placebo layer in hopper #2.
3. Place the powder for active layer in hopper #3.
4. Compress layer #1 tablets to desired weight (tablets for layer #1 should form a soft compact).
5. Compress layer #1 & Layer #2 tablets to desired combined weight of layer #1 and layer #2 weight (tablets should form a soft compact).
6. Compress the tri-layer tablet to the desired total tablet weight (layer #1 weight + layer #2 weight + layer #3 weight) Tablet should be at desired hardness.

A similar tablet of the invention is separately produced using the same top and bottom segments as the above, but using the following ingredients instead of Nu-Tab for the middle segment. The following are blended using a Patterson-Kelly "V" blender.

| Ingredients for middle segment: | Mg. |
|---|---|
| Dibasic calcium phosphate anhydrous | 158.59 |
| Magnesium stearate | 2.79 |
| PVP K-30 | 2.62 |
| Total | 164.00 |

### Manufacturing Instructions

1. Weigh each ingredient.
2. Screen each ingredient.
3. Place all of the ingredients, except the lubricant, into a suitable mixer and mix for desired time.
4. Add the lubricant to the blend from Step #3 and mix for desired time.
5. Add the blend to a suitable press fitted with the desired tooling and compress into tablets.

The tablets were compressed using oval 0.131 inch by 0.3222 inch, concave tablet punches to a hardness of 35 kilopound. The bottom segment was introduced first into the die. The tablet weight was 280 mg. Tablets with said middle segment were 6 mm high, and the inactive middle segment was 3.5-4 mm high.

### Tabletting Instructions

1. Place the powder for active layer in hopper #1.
2. Place the powder for placebo layer in hopper #2.
3. Place the powder for active layer in hopper #3.
4. Compress layer #1 tablets to desired weight (tablets for layer #1 should form a soft compact).
5. Compress layer #1 & Layer #2 tablets to desired combined weight of layer #1 and layer #2 weight (tablets should form a soft compact).
6. Compress the tri-layer tablet to the desired total tablet weight (layer #1 weight + layer #2 weight + layer #3 weight). Tablet should be at desired hardness.

In a similar way, other taller than wide tablets can be made on a tablet press, such as, the Korsch TRP900 which can produce taller tablets due to its design for deep filling cams which allow for deeper fills and greater distances between the upper and lower compression tools. To make an oval 0.131 inch by 0.3222 inch, concave tablet that is 12mm tall on the Korsch TRP900 the formulator would have to increase the weight of the inactive Nu-Tab® middle segment to about 323mg. Similarly to have a finished tablet height of 14mm the tablet would be formulated with a middle segment weighing about 388mg. If the formulator preferred, they could use the second example for a middle layer, i.e., the dibasic calcium phosphate (DCP) formulation. In such a case making an oval 0.131 inch by 0.3222 inch, concave tablet that is 12mm tall on the Korsch TRP900 the formulator would have to increase the weight of the inactive DCP middle segment to about 410mg. Similarly to have a finished tablet height of 14mm the tablet would be formulated with a middle segment weighing about 492mg.

The invention also includes the method of administering one or more drugs via the dosage forms such as tablets and tablettes of the invention to a patient, mammal, or other animal in need of pharmaceuticals for the prevention or treatment of an illness, maintenance of good health, retarding of aging, or other purpose. Included are methods of treating a patient with only one drug from a combination product, such as with a novel tablette of the invention, enabling downward dose adjustment for a variety of reasons; or, in a similar vein, a patient may be treated with one whole tablet containing a plurality of active drugs and in addition receive only one drug from a similar tablet, thus enabling upward dose adjustment. Combination products that can benefit from the invention, in which one drug is in an outer active segment, and a second and different drug is in the other outer active segment, and an inactive middle segment as in embodiments such as was described in paragraphs 3 and 4 above, include those containing the following pairs of drugs: amlodipine and either benazepril, chlorthalidone, or atorvastatin; benazepril and hydrochlorothiazide; olmesartan and hydrochlorothiazide; and many others, including the majority of the currently-produced combination products. Also included is the method of treating a patient with a precise partial dose of medication from a whole tablet, which may be a half or quarter of the whole dose, but may usefully be a different fraction. Warfarin especially may usefully be produced and dosed according to the invention with separable segments of the tablet that may but need not be as halves, quarters, etc. L-thyroxine and digoxin are other examples that could so benefit, along with warfarin.

The following give possible clinical situations in which the tablets of the invention could provide important benefits.
1. A currently marketed product in the United States is Caduet®, which contains the active ingredients atorvastatin calcium (atorvastatin) and amlodipine besylate (amlodipine) which are largely homogeneously interdispersed in an unscored tablet. The product is indicated to treat both hyperlipidemia (atorvastatin) and hypertension (amlodipine). A patient ingesting this tablet daily may then undergo a blood test and be diagnosed as having liver dysfunction as manifested by elevation of an enzyme's concentration in the blood. The physician may then recommend cessation, possibly temporary, of atorvastatin, which is stated by the manufacturer to be a possible cause of liver dysfunction. A patient receiving Caduet, however, would have to thus also discontinue amlodipine, which is not in this example desired by the physician. A tablet of the invention in which atorvastatin and amlodipine were segregated in different outer active segments, separated by a middle segment of adequate dimensions, would be a clear advance over the current Caduet formulation, because such a tablet would allow a patient to promptly continue ingesting amlodipine while stopping ingestion of atorvastatin, without having to go to a pharmacy and fill a new prescription for a tablet containing only amlodipine as the active ingredient, while having previously had the convenience of having both drugs combined in a single dosage form. The above embodiment of the invention represents an improvement over the current Caduet dosage form.
   Another clinical situation in which the invention is superior to Caduet is one in which a patient receiving amlodipine 5 mg once daily and atorvastatin 20 mg once daily is advised by a physician to increase the daily amlodipine dose to 10 mg once daily. A patient in possession of adequate tablets of the invention, with the active drugs segregated in a three-segment tablet, would be able to promptly increase the amlodipine dose by taking a whole tablet of the invention once daily, plus a tablette containing 5 mg of amlodipine, produced by breaking a second whole tablet of the invention.
   Another clinical situation in which the invention is superior to Caduet involves the case in which a physician wishes a patient to ingest atorvastatin 20 mg each morning and amlodipine 2.5 mg twice daily. The invention provides for amlodipine to be separated from atorvastatin and then broken precisely in half. The invention thus allows the patient the advantage of one tablet, whereas to accomplish this currently in the United States would require one 20 mg Lipitor® (atorvastatin) tablet and two Norvasc® (amlodipine) 2.5 mg tablets.
2. The combination of amlodipine besylate and benazepril hydrochloride (benazepril) is marketed in the United States under the brand name of Lotrel®. This product is a capsule that is routinely ingested whole. An embodiment of the invention provides a whole tablet containing one outer segment containing amlodipine as the only active drug and the other outer segment containing benazepril as the only active drug. If desired, either outer layer may be formed into more than one segment, as in Fig. 1a. As in example 1 above regarding Caduet, the middle segment is inactive and may be broken through to create two tablettes, each comprising a whole amount of each outer active segment plus approximately half of the amount of the middle inactive segment. If a patient were to develop a need for double the dose of one active drug but not the other, the tablet of the invention could meet that need. Alternatively, if a patient were to develop a need to ingest only one active drug, possibly temporarily, due to such conditions as blood pressure changes or a side effect to one drug but not the other, the tablet of the invention allows this to be done without a new dosage form being prescribed.
3. Another use of the invention involves the combination of amlodipine and chlorthalidone or another diuretic, which may usefully be combined to treat hypertension. Benefits of the invention are similar to those described in the paragraph immediately preceding this paragraph.
4. Another use of the invention involves the combination of olmesartan medoxomil (olmesartan, an angiotensin receptor blocker) and hydrochlorothiazide (HCTZ). This product is currently marketed in the United States under the name Benicar/HCT®, with the doses, respectively, of, in mg: 20/12.5, 40/12.5, and 40/25. A very common starting dose of a patient will be 20/12.5 once daily. The product is currently marketed in all strengths as a homogeneous tablet containing both active drugs. Formulated according to the current invention, a patient who begins treatment with the 20/12.5 dose may be increased with the same tablet to each of the other doses by ingesting one whole 20/12.5 tablet and either a half tablet containing 20 mg of olmesartan or a half tablet containing 25 mg of HCTZ. This will provide the physician an opportunity to investigate the new dose before giving the patient a new prescription. Other advantages of the invention are similar to those described above.
5. Another useful combination product that may be formulated according to the invention involves angiotensin converting enzyme inhibitors (ACEs) and diuretics such as HCTZ. Both types of drug not uncommonly have side effects, so that the invention will be useful to physicians in dealing with the side effects, as well as with changing dosing needs to deal with the anti-hypertensive and other clinical benefits of the drugs.
6. Another product that may benefit from the invention regarding separating active drugs in separate outer layers with an inactive middle segment (layer) is a combination product containing two active drugs, fluoxetine and olanzapine.

No limitation to the above therapeutic fields or to the specific examples within their fields is intended for tablets of the invention, which may be used in any suitable combination of drugs. No limitation to two-drug combinations exists, as well. For instance, one outer active segment of a tablet according to the invention could contain levodopa and carbidopa, and the other outer active segment could contain entacapone, a tablet product containing all three drugs in a homogeneous fashion that is currently marketed in the United States as Stalevo®. Also, a tablet per the invention could involve five layered segments, with, for example, amlodipine in one outer segment, an inactive segment adjoining it, a middle segment containing chlorthalidone or HCTZ, and a second inactive segment adjoining both it and the other outer segment that contains benazepril (see Fig. 8). If both inactive segments were of adequate dimensions to be conveniently breakable without damaging any of the three active segments, thus providing significant clinical advantages due to the adoption of flexible dosing of the different active segments.

The following list of possible combinations of a plurality of drugs is exemplary and not limiting. The combinations referred to may include two or more members of the classes listed. Drugs listed below, and herein, may for convenience exclude mention of any salt of a drug; e.g., "atorvastatin" is listed even though its marketed form is atorvastatin calcium.

Without limitation, useful combinations may include a plurality of drugs from within the following six drug classes. In addition, tablets of the invention may be created containing only one of a drug from the following list. With regards to combination use, two methods of use may apply to the invention. One of these methods is to place an individual drug in a granulation and a different individual drug (or combination of drugs) in a different granulation, potentially with an inactive granulation interposed between them; another method is to place a plurality of drugs in one or more segments.
1. Anti-anginal agents, for example:
   A. Calcium antagonists (see list below);
   B. Beta-blocker (see list below);
   C. Organic nitrate preparation (e.g., isosorbide mononitrate or dinitrate).
2. Anti-anginal agent plus an anti-platelet agent, such as aspirin, clopidogrel, or ticlopidine.
3. Two hypoglycemic agents (see list below).
4. Potassium chloride and any thiazide-type or loop diuretic (see lists below).
5. Lipid-lowering agent plus: hypoglycemic agent, anti-platelet agent, anti-anginal agent, and/or antihypertensive agent (see lists above and below)
   Hypoglycemic agents include: thiazolidinediones: pioglitazone, rosiglitazone; sulfonylureas: glyburide, glipizide, glimepiride, chlorpropamide;
   Biguanides: metformin;
   Meglitinides: nateglinide, repaglinide;
   Glucosidase inhibitors: acarbose, miglitol.
6. Antihypertensive agents:
   Beta-blockers: acebutolol, atenolol, bisoprolol, celiprolol, metoprolol, mebivolol, carvedilol (a mixed alpha-beta blocker), nadolol, oxprenolol, penbutolol, pindolol, propranolol, timolol, betaxolol, carteolol;
   Calcium antagonists (calcium-channel blockers): nifedipine, amlodipine, verapamil, diltiazem, nisoldipine, felodipine, isradipine, lacidipine, lercanidipine, nicardipine, manidipine;
   Thiazide-type diuretics (with or without potassium-retaining diuretics such as triamterene, amiloride, or spironolactone): hydrochlorothiazide, chlorothiazide, cyclopenthiazide, polythiazide, bendrofluazide, hydroflumethiazide, chlorthalidone, indapamide, methylclothiazide, metolazone;
   Angiotensin converting enzyme inhibitors: captopril, enalapril, lisinopril, ramipril, trandolapril, quinapril, perindopril, moexipril, benazepril, fosinopril;
   Angiotensin receptor blockers: losartan, valsartan, candesartan, telmisartan, eprosartan, irbesartan;
   High-ceiling (loop) diuretics (with or without potassium-retaining diuretics such as triamterene, amiloride, or spironolactone): furosemide, torsemide, ethacrynic acid, bumetamide;
   Aldosterone antagonist diuretics: spironolactone, eplerenone;
   Alpha-blockers: doxazosin, terazosin, prazosin, indoramin, labetolol (a mixed alpha-beta blocker);
   Central alpha-agonists: clonidine, methyldopa;
   Imidazoline: moxonidine;
   Direct vasodilators: hydralazine, minoxidil;
   Adrenergic neuronal blocker: guanethidine.
   Lipid-lowering agents include:
   Statins: lovastatin, simvastatin, pravastatin, rosuvastatin, atorvastatin, fluvastatin;
   Fibrates: clofibrate, bezafibrate, fenofibrate, gemfibrozil, ciprofibrate;
   Others: ezetimide, niacin, acipimox.

The combinations of drugs disclosed herein are for illustrative purposes and are not intended to limit the scope of the invention.

Regarding the important usage of the tablets and tablettes of the invention, that involving division of a tablet into tablettes containing similar active segments, most drugs that may undergo dosage adjustment will be preferred if they may be divided in an optimally precise manner. Examples of drugs that will especially benefit from the advances of the invention in this manner include narrow therapeutic index drugs such as warfarin, digoxin, L-thyroxine; vasoactive drugs such as amlodipine; hypoglycemic agents such as rosiglitazone and glipizide; and anxiolytics drugs such as alprazolam. These are however but a small fraction of the great mass of drugs that will benefit from the various embodiments and procedures of the invention.

There are numerous methods of use of the dosage forms of the invention, including its tablets and tablettes. Persons skilled in the medical and pharmaceutical arts will recognize the many advantages that the various embodiments of the invention allow over current products. Some examples of benefits of the inventions involving tablets containing exactly one similar active segment are described immediately below.
1. Warfarin is an anticoagulant marketed in the U.S. under the brand name Coumadin®, which is a scored tablet. Research has shown that patients do not break warfarin 5 mg tablets into equal 2.5 mg segments. The invention teaches different types of tablets that allow warfarin tablets of any common human dose to be broken into precise halves, and potentially precise thirds, quarters, etc. Thus a patient may utilize warfarin half-tablets produced as per the invention with similar confidence as in the whole tablet. Because warfarin doses are frequently broken, many clinical scenarios exist in which the invention will benefit patients.
2. Norvasc (amlodipine besylate or amlodipine herein) is marketed as unscored 2.5, 5, and 10 mg tablets in the U.S. These tablets are of irregular shape and are difficult to break. The FDA-approved dosage range is from 2.5 to 10 mg ingested orally daily. The invention allows improved functionality of amlodipine. For example, under the invention, a patient receiving 5 mg daily who a physician wishes to increase to 7.5 mg daily may simply utilize a tablet of the invention that comprises two separate 2.5 mg segments to increase the dose to precisely 7.5 mg, such as by ingesting one whole 5 mg tablet and one 2.5 mg tablette created by breaking a 5 mg tablet into two tablettes each containing 2.5 mg of amlodipine. Convenience and cost savings are clear. Similarly, a patient receiving a 10 mg dose of Norvasc who is advised to reduce the dose to 5 mg daily must currently purchase a new prescription for 5 mg Norvasc tablets. The invention provides the ability to provide a 10 mg tablet that may be broken into two tablettes, each containing precisely 5 mg of amlodipine. The invention may therefore enable greater flexibility of treating patients, and provide cost savings as well. A further benefit of the invention is that various embodiments allow fully accurate separation of a tablet into a tablette comprising one-fourth of the dose of the active ingredient as is found in the whole tablet. This may for example be done for amlodipine by providing four active segments all containing 2.5 mg of amlodipine and all contiguous with the same side of an inactive outer segment (see embodiment #1; and see Fig. 6a modified to have four and not two active segments). Thus, a 10 mg amlodipine tablet of the invention may be utilized to provide a 7.5 mg dose; or, it may be utilized to provide four 2.5 mg doses.

A further benefit of the invention may relate to pediatric or geriatric doses, which may not be produced in appropriate dose strengths. In the case of amlodipine, a 1.25 mg daily dose may be useful in either small children with hypertension, or in frail elderly patients with angina or hypertension, who may have hepatic dysfunction. Even though the United States Food and Drug Administration (FDA) has not approved a 1.25 mg dose, precise divisibility of the approved 2.5 mg dose would allow a 1.25 mg daily dose. In addition, precise divisibility of the approved 2.5 mg dose will allow accurate dosing of 3.75 mg daily.

Another use of the invention is to for the first time enable a method of cost savings to insurers and patients. The invention allows this because many drugs, such as Norvasc and Coumadin, have pricing that differs little (if at all) between different doses. Because tablet splitting is imprecise for most scored tablets, the practice of mandatory splitting has been met with disapproval by most physician and pharmacist organizations. The invention enables tablet splitting due to provide accurate dosing when a tablet (or some tablettes, as in Fig. 1b) of the invention are broken as described herein. Substantial benefits are foreseen from this innovation. In addition, the ability to separate one active drug from another in a combination product has cost saving advantages, as well.

## Claims

1. A segmented layered pharmaceutical taller than wide tablet obtainable by sequential introduction of granulations into a tablet die, having a separation mark positioned within a segment which is not a top or bottom segment, and which lacks a pharmacologically active quantity of any drug; such that on application of force the tablet will break through the inactive part of the tablet, wherein the separation mark or marks is/are selected from the group consisting of one or more of the following:
(a) a score located in a side wherein said score is not oriented vertically;
(b) indicia on at least one side that locates a desired breaking region of said tablet;
(c) a band which is located on one segment.

2. A pharmaceutical tablet as defined in claim 1 where the separation mark is located entirely within one segment.

3. A pharmaceutical tablet as defined in claim 1 where the separation mark is in a pharmaceutical tablet comprising three segments where the separation mark is placed on or in a single segment which is not a top or bottom segment.

4. A pharmaceutical tablet as defined in claim 3 where at least one additional separation mark is placed in one or more segments which is a top or bottom segment.

5. A pharmaceutical tablet as defined in claim 1 wherein the separation mark comprises a score.

6. A pharmaceutical tablet as defined in claim 5 in which said separation mark that comprises a score is substantially horizontally oriented on said tablet.

7. A pharmaceutical tablet in claim 5 in which said separation mark that comprises a score is substantially confined within one segment.

8. A pharmaceutical tablet as defined in claim 1 wherein the separation mark comprises indicia.

9. A pharmaceutical tablet as defined in claim 1 wherein the separation mark comprises printed indicia.

10. A pharmaceutical tablet as defined in claim 1 wherein the separation mark comprises printed indicia that comprise a printed line or lines, a printed logo or logos, a printed arrow or arrows, a numerical or other type of dose indication or indications or a code number or numbers.

11. A pharmaceutical tablet as defined in claim 1 wherein the separation mark comprises a plurality of scores.

12. A pharmaceutical tablet as defined in claim 1 where the separation mark is in a pharmaceutical tablet having three vertically disposed segments where the separation mark is placed solely in the inner segment which is interposed between the top and bottom segments, said separation mark being placed horizontally on the tablet, wherein the horizontal axis corresponds to the horizontal axis of a tablet die in which said tablet is compressed.

13. A pharmaceutical tablet as defined in claim 3 where the separation mark is in a pharmaceutical tablet having three vertically disposed contiguous segments where the separation mark is placed in a single segment which is not a top or bottom segment, said top segment and said bottom segment containing the same or different drugs.

14. A method of breaking a pharmaceutical tablet as defined in claim 1 which comprises breaking said tablet at or by said separation mark.

15. A method of breaking a pharmaceutical tablet as defined in claim 1 by breaking through said tablet between two or more separation marks that may include printed lines, printed arrows, or surface.

16. A pharmaceutical compressed tablet as defined in claim 1 for the administration of a partial dose of a drug to a subject in need, wherein said drug is pharmacologically effective in the treatment of cardiovascular conditions, psychiatric conditions, diabetes, thyroid disorders, pain or thrombotic disorders, and wherein said partial dose is obtained by breaking said pharmaceutical tablet at or as guided by said separation mark to form two or more tablettes.

17. A pharmaceutical tablet as defined in claim 1 in which said drug or drugs is or are pharmacologically effective in the treatment of cardiovascular conditions, psychiatric conditions, diabetes, thyroid disorders, pain or thrombotic disorders.

## Patentansprüche

1. Segmentierte, höhere als breite pharmazeutische Schichttablette, die durch die aufeinanderfolgende Eingabe von Granulationen in eine Tablettenpressform erhalten werden kann und die eine Trennmarkierung aufweist, die in einem Segment angeordnet ist, das kein oberes oder unteres Segment ist und das keine pharmakologisch wirksame Menge irgendeines Wirkstoffs aufweist; derart, dass die Tablette bei Aufbringung einer Kraft durch den nicht wirksamen Teil der Tablette gebrochen wird, wobei die Trennmarkierung oder -markierungen aus der Gruppe ausgewählt ist bzw. sind, die aus einem oder mehreren von Folgendem besteht:
(a) einer Kerbe, die sich in einer Seite befindet, wobei diese Kerbe nicht senkrecht ausgerichtet ist;
(b) Zeichen auf mindestens einer Seite, die einen gewünschten Bruchbereich dieser Tablette bestimmen;
(c) einem Band, das sich auf einem Segment befindet.

2. Pharmazeutische Tablette nach Anspruch 1, wobei sich die Trennmarkierung vollständig innerhalb eines Segments befindet.

3. Pharmazeutische Tablette nach Anspruch 1, wobei die Trennmarkierung in einer pharmazeutischen Tablette ist, die drei Segmente umfasst, wobei die Trennmarkierung auf oder in einem einzigen Segment angeordnet ist, das kein oberes oder unteres Segment ist.

4. Pharmazeutische Tablette nach Anspruch 3, wobei mindestens eine zusätzliche Trennmarkierung in einem oder mehreren Segmenten angeordnet ist, bei dem bzw. denen es sich um ein oberes oder unteres Segment handelt.

5. Pharmazeutische Tablette nach Anspruch 1, wobei die Trennmarkierung eine Kerbe umfasst.

6. Pharmazeutische Tablette nach Anspruch 5, wobei die Trennmarkierung, die eine Kerbe umfasst, im Wesentlichen waagrecht auf dieser Tablette ausgerichtet ist.

7. Pharmazeutische Tablette nach Anspruch 5, wobei die Trennmarkierung, die eine Kerbe umfasst, im Wesentlichen innerhalb eines Segments begrenzt ist.

8. Pharmazeutische Tablette nach Anspruch 1, wobei die Trennmarkierung Zeichen umfasst.

9. Pharmazeutische Tablette nach Anspruch 1, wobei die Trennmarkierung gedruckte Zeichen umfasst.

10. Pharmazeutische Tablette nach Anspruch 1, wobei die Trennmarkierung gedruckte Zeichen umfasst, die eine gedruckte Linie oder gedruckte Linien, ein gedrucktes Logo oder gedruckte Logos, einen gedruckten Pfeil oder gedruckte Pfeile, eine numerische oder andersartige Dosisangabe oder numerische oder andersartige Dosisangaben oder eine Kennzahl oder Zahlen umfassen.

11. Pharmazeutische Tablette nach Anspruch 1, wobei die Trennmarkierung eine Vielzahl von Kerben umfasst.

12. Pharmazeutische Tablette nach Anspruch 1, wobei die Trennmarkierung in einer pharmazeutischen Tablette ist, die drei senkrecht angeordnete Segmente aufweist, wobei die Trennmarkierung nur im inneren Segment angeordnet ist, das zwischen das obere und das untere Segment eingefügt ist, wobei diese Trennmarkierung waagrecht auf der Tablette angeordnet ist, wobei die waagrechte Achse der waagrechten Achse der Tablettenpressform entspricht, in der die Tablette verpresst wird.

13. Pharmazeutische Tablette nach Anspruch 3, wobei die Trennmarkierung in einer pharmazeutischen Tablette ist, die drei senkrecht angeordnete benachbarte Segmente aufweist, wobei die Trennmarkierung in einem einzigen Segment angeordnet ist, das kein oberes oder unteres Segment ist, wobei das obere Segment und das untere Segment die gleichen oder verschiedene Wirkstoffe enthalten.

14. Verfahren zum Brechen einer pharmazeutischen Tablette nach Anspruch 1, welches das Brechen diese Tablette an oder durch die Trennmarkierung umfasst.

15. Verfahren zum Brechen einer pharmazeutischen Tablette nach Anspruch 1 durch Durchbrechen dieser Tablette zwischen zwei oder mehr Trennmarkierungen, die gedruckte Linien, gedruckte Pfeile oder eine Fläche umfassen können.

16. Verpresste pharmazeutische Tablette nach Anspruch 1 zum Verabreichen einer Teildosis eines Wirkstoffs an eine bedürftige Person, wobei dieser Wirkstoff in der Behandlung von kardiovaskulären Leiden, psychiatrischen Leiden, Diabetes, Schilddrüsenerkrankungen, Schmerzen oder thrombotischen Erkrankungen pharmakologisch wirksam ist, und wobei diese Teildosis erhalten wird, indem die pharmazeutische Tablette an oder anhand der Trennmarkierung durchgebrochen wird, um zwei oder mehr Tabletten zu bilden.

17. Pharmazeutische Tablette nach Anspruch 1, bei welcher der Wirkstoff oder die Wirkstoffe bei der Behandlung von kardiovaskulären Leiden, psychiatrischen Leiden, Diabetes, Schilddrüsenerkrankungen, Schmerzen oder thrombotischen Erkrankungen pharmakologisch wirksam ist bzw. sind.

## Revendications

1. Un comprimé pharmaceutique à couches segmenté plus haut que large pouvant être obtenu par introduction séquentielle de granulations dans une matrice à comprimé, ayant une marque de séparation positionnée dans un segment qui n'est pas un segment supérieur ou inférieur, et qui est dépourvu d'une quantité pharmacologiquement active de n'importe quel médicament ; de manière que, lors de l'application d'une force, le comprimé se casse à travers la partie inactive du comprimé, dans lequel la marque ou les marques de séparation est ou sont sélectionnées à partir du groupe comprenant un ou plusieurs des éléments suivants :
(a) un sillon situé dans un côté dans lequel ledit sillon n'est pas orienté verticalement ;
(b) des indices sur au moins un côté qui localisent une zone de rupture désirée dudit comprimé ;
(c) une bande qui est située sur un segment.

2. Un comprimé pharmaceutique selon la revendication 1, dans lequel la marque de séparation est située entièrement à l'intérieur d'un segment.

3. Un comprimé pharmaceutique selon la revendication 1, dans lequel la marque de séparation se trouve dans un comprimé pharmaceutique comprenant trois segments où la marque de séparation est placée sur ou dans un unique segment qui n'est pas un segment supérieur ou inférieur.

4. Un comprimé pharmaceutique selon la revendication 3, dans lequel au moins une marque de séparation additionnelle est placée dans un ou plusieurs segments qui est un segment supérieur ou inférieur.

5. Un comprimé pharmaceutique selon la revendication 1, dans lequel la marque de séparation comprend un sillon.

6. Un comprimé pharmaceutique selon la revendication 5, dans lequel ladite marque de séparation qui comprend un sillon est orientée sensiblement horizontalement sur ledit comprimé.

7. Un comprimé pharmaceutique selon la revendication 5, dans lequel ladite marque de séparation qui comprend un sillon est sensiblement confinée à l'intérieur d'un segment.

8. Un comprimé pharmaceutique selon la revendication 1, dans lequel la marque de séparation comprend des indices.

9. Un comprimé pharmaceutique selon la revendication 1, dans lequel la marque de séparation comprend des indices imprimés.

10. Un comprimé pharmaceutique selon la revendication 1, dans lequel la marque de séparation comprend des indices imprimés qui comprennent une ou plusieurs lignes imprimées, un ou plusieurs logos imprimés, une ou plusieurs flèches imprimées, une ou plusieurs indications de dose numériques ou d'un autre type ou un ou plusieurs numéros de code.

11. Un comprimé pharmaceutique selon la revendication 1, dans lequel la marque de séparation comprend une pluralité de sillons.

12. Un comprimé pharmaceutique selon la revendication 1, dans lequel la marque de séparation se trouve dans un comprimé pharmaceutique comprenant trois segments disposés verticalement où la marque de séparation est placée uniquement dans le segment intérieur qui est interposé entre les segments supérieur et inférieur, ladite marque de séparation étant placée horizontalement sur le comprimé, dans lequel l'axe horizontal correspond à l'axe horizontal d'une matrice à comprimé dans laquelle ledit comprimé est compressé.

13. Un comprimé pharmaceutique selon la revendication 3, dans lequel la marque de séparation se trouve dans un comprimé pharmaceutique comprenant trois segments contigus disposés verticalement où la marque de séparation est placée dans un unique segment qui n'est pas un segment supérieur ou inférieur, ledit segment supérieur et ledit segment inférieur contenant les mêmes médicaments ou des médicaments différents.

14. Un méthode de rupture d'un comprimé pharmaceutique selon la revendication 1, qui comprend la rupture dudit comprimé au niveau ou par ladite marque de séparation.

15. Un méthode de rupture d'un comprimé pharmaceutique selon la revendication 1 par rupture dudit comprimé entre au moins deux marques de séparation qui peuvent comprendre des lignes imprimées, des flèches imprimées ou une surface.

16. Un comprimé pharmaceutique compressé selon la revendication 1 pour l'administration d'une dose partielle d'un médicament à un sujet qui en a besoin, dans lequel ledit médicament est pharmacologiquement efficace dans le traitement de conditions cardiovasculaires, de conditions psychiatriques, de diabètes, de troubles de la thyroïde, de la douleur ou de troubles thrombotiques, et dans lequel ladite dose partielle est obtenue par rupture dudit comprimé pharmaceutique au niveau de ou comme guidé par ladite marque de séparation pour former deux ou plusieurs comprimés.

17. Un comprimé pharmaceutique selon la revendication 1, dans lequel ledit médicament ou lesdits médicaments est ou sont pharmacologiquement efficaces dans le traitement de conditions cardiovasculaires, de conditions psychiatriques, de diabètes, de troubles de la thyroïde, de la douleur ou de troubles thrombotiques.
